(19) Europäisches Patentamt
European Patent Office
Office européen des brevets

(11) Numéro de publication: **0 115 997**
**B1**

# (12) FASCICULE DE BREVET EUROPÉEN

(45) Date de publication du fascicule du brevet: **22.07.87**

(51) Int. Cl.⁴: **C 07 C 149/23**, C 07 C 149/41, C 07 C 153/09, C 07 D 213/75, C 07 D 235/30, C 07 D 277/00, A 61 K 31/16, A 61 K 31/265, A 61 K 31/41, A 61 K 31/44

(21) Numéro de dépôt: **84400216.2**

(22) Date de dépôt: **01.02.84**

(54) **Nouveaux dérivés de omega-mercaptopropanamide et de ses homologues, leur procédé de préparation, leur application comme médicaments, les compositions les renfermant et les nouveaux intermédiaires obtenus.**

(30) Priorité: **07.02.83 FR 8301862**

(43) Date de publication de la demande: **15.08.84 Bulletin 84/33**

(45) Mention de la délivrance du brevet: **22.07.87 Bulletin 87/30**

(84) Etats contractants désignés: **AT BE CH DE FR GB IT LI LU NL SE**

(56) Documents cité:
**EP-A-0 001 989**
**EP-A-0 066 956**
**US-A-4 053 651**
**US-A-4 148 815**

(73) Titulaire: **ROUSSEL- UCLAF, 35, boulevard des Invalides, F-75007 Paris (FR)**

(72) Inventeur: **Vevert, Jean- Paul F., 55, rue Rouget de L'Isle, F-93500 Pantin (FR)**
Inventeur: **Delevallée, Françoise F., 55, rue Diderot, F-94320 Vincennes (FR)**
Inventeur: **Deraedt, Roger F., 23, allée J.B. Clément, F-93320 Pavillons- sous- Bois (FR)**

(74) Mandataire: **Bourgouin, André, Département des Brevets ROUSSEL UCLAF B.P. no 9, F-93230 Romainville (FR)**

**EP 0 115 997 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

## Description

La présente invention concerne de nouveaux dérivés de ω- mercaptopropanamide et de ses homologues, leur procédé de préparation, leur application comme médicaments, les compositions les renfermant et les nouveaux intermédiaires obtenus.

Le brevet européen 1989 décrit des dérivés du mercaptopropanamide et notamment des dérivés portant une fonction carbonyle reliée à la fonction amide. Ces produits sont présentés comme des immunorégulateurs utiles dans de très nombreuses maladies mais ne sont pas décrits comme analgésiques.

Le brevet US 4.148.815 décrit un procédé d'extraction de métaux utilisant le N-phényl 2 mercapto acétamide et ses homologues.

Le brevet US 4.053.651 décrit des dérivés mercapto propanamide d'acides aminés qui sont des inhibiteurs de l'enzyme de conversion de l'angiotensine.

Le brevet européen 066956 décrit des inhibiteurs d'enképhalinase dont le substituant porté par la fonction amide est très différent de celui des produits de la présente demande.

L'invention a pour objet les composés de formule (I):

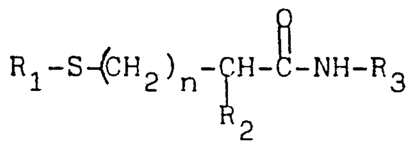 (I)

dans laquelle $R_1$ représente un atome d'hydrogène ou un radical

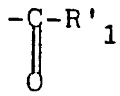 ,

$R'_1$ étant un radical alcoyle renfermant de 1 à 5 atomes de carbone ou un radical aryle éventuellement substitué par un radical hydroxy, un radical alcoyle renfermant de 1 à 5 atomes de carbone, un radical alcoxy renfermant de 1 à 5 atomes de carbone, le radical nitro ou un atome d'halogène, n représente un nombre entier pouvant varier de 1 à 5, $R_2$ représente un atome d'hydrogène, un radical alcoyle renfermant de 1 à 5 atomes de carbone, un radical aryle ou arylalcoyle renfermant de 6 à 15 atomes de carbone, éventuellement substitués par un radical alcoyle renfermant de 1 à 5 atomes de carbone, un radical alcoxy renfermant de 1 à 5 atomes de carbone, un radical hydroxyle, un atome d'halogène ou un radical trifluorométhyle, $R_3$ représente un radical hétérocyclique choisi parmi les radicaux thiazolyle, 4,5-dihydrothiazolyle, pyridinyle, oxazolyle, isoxazolyle, imidazolyle, pyrimidyle, tétrazolyle, benzimidazolyle, benzothiazolyle ou benzoxazolyle, éventuellement substitué par un radical alcoyle renfermant de 1 à 5 atomes de carbone ou $R_3$ représente un atome d'hydrogène, un radical phényle éventuellement substitué par un ou plusieurs radicaux choisis dans le groupe constitué par les radicaux alcoyles renfermant de 1 à 5 atomes de carbone, les radicaux alcoxy renfermant de 1 à 5 atomes de carbone, le radical hydroxyle, le radical nitro, les atomes d'halogènes, le radical trifluorométhyle, le radical carboxyméthyle, les radicaux alcoxy carbonyl méthyle dans lesquels le radical alcoxy renferme de 1 à 5 atomes de carbone, les radicaux aralcoyloxy renfermant de 7 à 15 atomes de carbone et les radicaux:

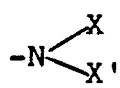 ,

dans lesquels X et X', identiques ou différents, représentent un atome d'hydrogène ou un radical alcoyle renfermant de 1 à 5 atomes de carbone, $R_3$ ne pouvant toutefois représenter un radical phényle non substitué ou substitué par un ou plusieurs radicaux choisis dans le groupe constitué par les radicaux alcoyles renfermant de 1 à 5 atomes de carbone, les radicaux alcoxy renfermant de 1 à 5 atomes de carbone et les atomes d'halogènes, lorsque $R_2$ représente un atome d'hydrogène et lorsque n est égal à 1 ou à 2 et $R_3$ ne pouvant représenter un radical phényle substitué par un radical carboxyméthyle lorsque $R_2$ représente un radical méthyle et lorsque n est égal à 1, ainsi que leurs sels d'addition avec les acides et les bases.

Lorsque $R_1$ représente un radical

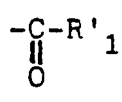 ,

$R'_1$ est de préférence un radical méthyle ou éthyle.

Lorsque $R'_1$ est un radical aryle, il s'agit de préférence d'un radical phényle.

Lorsque $R'_1$ est un radical aryle substitué, il s'agit de préférence d'un radical aryle substitué par un radical choisi dans le groupe formé par le radical hydroxy, les radicaux méthyle et éthyle, les radicaux méthoxy et éthoxy, le radical nitro et l'atome de chlore.

n est de préférence égal à 1 ou à 2.

Lorsque $R_2$ représente un radical alcoyle, il s'agit de préférence du radical méthyle, éthyle, n-propyle, n-butyle, n-pentyle, isopropyle ou isobutyle.

Lorsque $R_2$ est un radical aryle, il s'agit de préférence d'un radical phényle.

Lorsque $R_2$ est un radical arylalcoyle, il s'agit de préférence d'un radical benzyle ou phénéthyle.

Lorsque $R_2$ est un radical aryle ou arylalcoyle substitué, il s'agit de préférence d'un radical aryle ou arylalcoyle substitué par un radical méthyle ou éthyle, un radical méthoxy ou éthoxy, un radical hydroxyle, un atome de chlore ou un radical trifluorométhyle.

Lorsque $R_3$ représente un radical hétérocyclique substitué par un radical alcoyle, il s'agit de préférence d'un radical hétérocyclique substitué par un radical métbyle ou étbyle.

Lorsque $R_3$ représente un radical phényle substitué, il s'agit de préférence d'un radical phényle substitué par un ou plusieurs radicaux choisis dans le groupe formé par le radical hydroxy, les radicaux méthyle et éthyle, les radicaux méthoxy et éthoxy, le radical trifluorométhyle, le radical nitro, l'atome de chlore, les radicaux amino et diméthylamino, les radicaux carboxy méthyle et alcoxy carbonylméthyle et le radical benzyloxy.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique glyoxylique, aspartique, alcane sulfoniques tels que les acides méthane et éthane sulfoniques, arylsulfoniques, tels que les acides benzène et paratoluène sulfoniques et arylcarboxyliques.

Parmi les sels formés avec les bases, on peut citer les sels de métaux alcalins comme le sodium ou le potassium et les sels d'amines, par exemple, de triméthylamine ou de diméthylamine.

L'invention a notamment pour objet les composés de formule (I) pour lesquels $R_1$ est un atome d'hydrogène, ainsi que leurs sels d'addition avec les acides et les bases et ceux pour lesquels $R_1$ est un radical acétyle, ainsi que leurs sels d'addition avec les acides et les bases.

L'invention a plus particulièrement pour objet les composés de formule (I) pour lesquels $R_2$ ne représente pas un atome d'hydrogène et notamment ceux pour lesquels n = 1 et $R_2$ est un radical benzyle, ainsi que leurs sels d'addition avec les acides et les bases et tout particulièrement ceux pour lesquels $R_3$ est un radical phényle éventuellement substitué par un ou plusieurs atomes de chlore ou par un ou plusieurs radicaux méthoxy ou un radical pyridinyle, anisi que leurs sels d'addition avec les acides et les bases.

Parmi les composés de l'invention, on peut citer les produits cités dans les exemples et tout particulièrement:

- l'α-(mercaptométhyl)N-phényl benzène propanamide,
- l'éthanethioate de S-/ 3-oxo 3-(phénylamino)2-(phénylméthyl) propyle/,
- l'α-(mercaptométhyl)N-4-méthoxyphényl)benzène propanamide,
- l'α-(mercaptométhyl)N-(4-méthoxyphényl)benzène propanamide,
- l'α-(mercaptométhyl)N-(4-pyridinyl)benzène propanamide,

ainsi que leurs sels d'addition avec les acides et les bases.

L'invention a également pour objet un procédé de préparation des produits de formule (I), dans laquelle $R_1$, $R_2$ et $R_3$ ont les significations données précédemment et dans laquelle n = 1, caractérisé en ce que l'on soumet un acide de formule (II):

$$R'_1-\overset{\overset{\displaystyle O}{\|}}{C}-S-CH_2-\underset{\underset{\displaystyle R_2}{|}}{CH}-COOH \qquad (II)$$

dans laquelle $R'_1$ et $R_2$ ont les significations déjà indiquées, ou un dérivé fonctionnel de cet acide, à l'action d'un produit de formule (III):

$$H_2N-R_3 \qquad (III)$$

dans laquelle $R_3$ a la signification déjà indiquée, pour obtenir un produit de formule (I), dans laquelle $R_1$ =

$$R'_1-\underset{\underset{\displaystyle O}{\|}}{C}-,$$

$R'_1$ ainsi que $R_2$ et $R_3$ ayant les significations déjà indiquées et dans laquelle n = 1, produit de formule (I), que le cas échéant, l'on saponifie, pour obtenir un produit de formule (I) dans laquelle $R_1$ représente un atome d'hydrogène, produits de formule (I) que l'on soumet si désiré, à l'action d'une base ou d'un acide pour en former le sel.

L'invention concerne aussi un procédé de préparation des produits de formule (I), dans laquelle $R_1$, $R_2$ et $R_3$ ont les significations données précédemment et dans laquelle n représente un nombre entier pouvant varier de 1 à 5, caractérisé en ce que l'on soumet un acide de formule (IV):

$$X_1-(CH_2)_n-\overset{R_2}{\overset{|}{C}}H-COOH \qquad (IV)$$

dans laquelle $X_1$ est un atome d'halogène, et dans laquelle n et $R_2$ ont les significations données ci-dessus ou un dérivé fonctionnel de celui-ci, à l'action d'un produit de formule (III) ($NH_2$-$R_3$) précédent, pour obtenir un produit de formule (V):

$$X_1-(CH_2)_n-\overset{R_2}{\overset{|}{C}}H-COHN-R_3 \qquad (V)$$

dans laquelle $X_1$ n, $R_2$ et $R_3$ ont les significations précédentes, que l'on soumet à l'action de l'anion d'un thioacide de formule (VI):

$$R'_1-\underset{\underset{O}{\|}}{C}-SH \qquad (VI)$$

dans laquelle $R'_1$ conserve sa signification pour obtenir un produit de formule (I) dans laquelle $R_1$ =

$$R'_1-\underset{\underset{O}{\|}}{C}- \quad ,$$

n représente un nombre entier pouvant varier de 1 à 5, et dans laquelle $R'_1$, $R_2$ et $R_3$ ont les significations données ci-dessus, produit de formule (I) que le cas échéant l'on saponifie, pour obtenir un produit de formule (I) dans laquelle $R_1$ représente un atome d'hydrogène, produits de formule (I) que l'on soumet, si désiré, à l'action d'un acide ou d'une base pour en former le sel.

Les dérivés fonctionnels des acides de formule (II) et (IV) sont de préférence des chlorures d'acide.

Dans le produit de formule (IV), $X_1$ est de préférence un atome de chlore.

Les réactions de condensation entre les produits de formules (II) ou (IV) et les produits de formule (III) s'effectuent au sein d'un solvant tel que l'éther, le tétrahydrofurane, un solvant chloré, notamment le chlorure de méthylène le 1,1-dichloroéthane, le chloroforme ou le tétrachlorure de carbone, une cétone, notamment l'acétone ou la méthyléthylcétone.

Les composés de formule (I) tels que définis ci-dessus, ainsi que leurs sels d'addition avec les acides et les bases, présentent d'intéressantes propriétés pharmacologiques. Ils présentent, en particulier, de très intéressantes propriétés inhibitrices de l'enképhalinase et sont doués d'une très bonne activité analgésique.

L'enképhalinase est une dipeptidylcarboxypeptidase qui hydrolyse spécifiquement la méthionine et la leucine enképhaline entre le 3ème et le 4ème acide aminé, libérant ainsi un tripeptide Tyr-Gly-Gly (Swerts, J.P., Perdrisot, R., Patey, G.; De La Baume, S., and Scbwartz, J.C., Europ. J. Pharmacol., 1979, 57, 279).

L'enképhalinase participe ainsi directement à la dégradation physiologique des enképhalines, ligands naturels endogènes des récepteurs opiacés. Les composés de l'invention, qui retardent la dégradation des enképhalines, stimulent donc les réactions de défense de l'organisme contre la douleur.

Ces propriétés justifient leur application en thérapeutique et l'invention a également pour objet, à titre de médicaments, les produits tels que définis par la formule (I) ci-dessus, ainsi que les sels d'addition avec les acides et les bases pharmeceutiquement acceptables desdits produits de formule (I).

La présente invention a tout particulièrement pour objet, à titre de médicaments, l'α-(mercaptométhyl) N-phényl benzène propanamide, l'éthanethioate de S-/ 3-oxo 3-(phénylamino)2-(phénylmethyl)propyle/, l'α-(mercaptométhyl)N-(3-méthoxy phényl)benzène propanamide, l'α-(mercaptométhyl)N-(4-méthoxy phényl)benzène propanamide et l'α-(mercaptométhyl)N-(4-pyridinyl)benzène propanamide ainsi que leurs sels d'addition avec les acides et les bases pharmaceutiquement acceptables.

Les médicaments, objet de l'invention, peuvent être utilisés dans le traitement des algies musculaires, articulaires ou nerveuses, des affections rhumatismales, des douleurs dentaires, des zonas et des migraines, ainsi que dans le traitement des maladies inflammatoires, notamment des arthroses, des lumbagos, et aussi à titre de traitement complémentaire dans les états infectieux et fébriles.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, las granulés, les suppositoires, les préparations injectables, les pommades, les crèmes, les gels et les préparations en aérosols; elles sont préparées selon les méthodes usuellas. Le principe actif peut y être

incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 mg et 2 g de principe actif, par jour, par voie orale.

Certains des composés de formule (II) et (IV) utilisés comme produits de départ d'un des procédés de l'invention sont décrits et préparés dans le brevet Ondetti et al. US 4 053 651.

Les produits de formule (II) et (IV) non décrits dans ce brevet peuvent être préparés comme indiqué dans ce brevet.

Les composés de formule (V) sont des produits chimiques nouveaux; l'invention a donc pour objet, ces produits à titre de produits industriels nouveaux, notamment à titre de produits intermédiaires nécessaires à la mise en oeuvre du procédé de l'invention.

Les exemples suivants illustrent l'invention sans toutefois la limiter.

**Exemple 1**:

Ethanethioate de S/ 3-oxo-3(phénylamino)2-(phénylméthyl)propyle/.

Stade A: Préparation du chlorure d'α-(acétylthiométhyl) benzène propanoyle

On dissout 1,21 g d'acide α-benzyl acrylique (produit décrit et préparé selon C. Mannich et Coll.Chem.Ber. 57B, 1116-8, (1924)) dans 0,8 g d'acide thioacétique, garde une heure à température ambiante sous argon, chauffe une heure à 100° C, élimine l'excès d'acide thioacétique sous pression réduite. A l'huile résiduelle, on ajoute sous agitation sous argon et à 0° C, goutte à goutte, 1,5 g de chlorure de thionyle. On garde une nuit à température ambiante, élimine l'excés de chlorure de thionyle sous pression réduite, ajoute 50 cm3 de benzène que l'on élimine ensuite sous pression réduite. Après distillation de l'huile résiduelle, on obtient 1,87 g de produit attendu.

Eb. = 150° C ($10^{-2}$ Torr).

Stade B: Ethanethioate de S-/ 3-oxo-3-(phénylamino) 2-(phénylméthyl)propyle/

On ajoute sous argon 4,2 g d'aniline en solution dans 250 $cm^3$ de dichlorométhane dans une solution refroidie vers -35° C de 16 g de produit préparé au stade A dans 250 $cm^3$ de dichlorométhane, on agite une nuit à température ambiante, élimine par filtration les cristaux de chlorhydrate d'aniline formés, lave le filtrat à l'acide chlorhydrique 0,1 N puis à l'eau jusqu'à neutralité, sèche, élimine les solvants. On obtient 21 g de produit brut auquel on ajoute un mélange de 100 $cm^3$ de pentane et de 50 $cm^3$ d'éther, essore, sèche sous pression réduite et obtient 16 g de produit attendu fondant à 110° C.

**Exemple 2**:

α-(mercaptométhyl)N-phényl benzène propanamide.

On ajoute entre 5 et 10° C, sous argon, 20 $cm^3$ de soude normale à 6 g de produit de l'exemple 1 dans 100 $cm^3$ de méthanol, agite pendant sept heures 30 minutes à température ambiante, élimine le méthanol sous pression réduite, filtre, acidifie le filtrat par de l'acide chlorhydrique N, extrait au dichlorométhane.

On recueille 3,5 g d'une huile que l'on purifie par chromatographie sur silice en éluant avec du pentane à 50 % d'éther. On obtient 2,8 g d'un produit brut qui est trituré dans le pentane, filtré et séché sous pression réduite. On obtient 2,4 g de produit attendu fondant à 70° C.

**Exemple 3**:

Ethanethioate de S-/3-(4-chlorophénylamino)3-oxo 2-(phénylméthyl)propyle/

On ajoute, goutte à goutte, sous argon et sous agitation, 8,05 g de 4-chloroaniline dans 150 $cm^3$ de dichlorométhane à une solution maintenue vers -35° C de 8 g de produit préparé au stade a de l'exemple 1 dans 100 $cm^3$ de dichlorométhane.

La température remonte jusqu'à 20° C, on agite 64 heures, élimine par filtration les cristaux de chlorhydrate de 4-chloro-aniline, lave le filtrat par de l'acide chlorhydrique 0,1N puis à l'eau. On sèche, élimine les solvants, obtient 10,6 g de produit brut que l'on triture dans 150 $cm^3$ d'un mélange pentaneéther éthylique (1-2). On filtre, sèche sous pression réduite et obtient 6,9 g de produit attendu fondant à 130° C.

**Exemple 4**:

N-(4-chlorophényl) α-(mercaptométhyl)benzène propanamide.

On ajoute sous argon en 30 minutes et vers +5°C, 33 $cm^3$ de soude normale à 11,2 g de produit de l'exemple 3 en solution dans 300 $cm^3$ de méthanol. On agite 2 heures à +15°C, redescend à + 2°C et amène le pH à neutralité avec de l'acide chlorhydrique N. On ajoute 200 $cm^3$ d'eau glacée, essore les cristaux, les lave à l'eau et les sèche sous pression réduite. On obtient 8,9 g de produit brut que l'on chromatographie sur silice en éluant par del l'hexane à 50 % de dichlorométhane. On recueille 6,4 g de produit que l'on triture dans du pentane, filtre et sèche sous pression réduite. On obtient 6,2 g de produit attendu fondant à 142°C.

En opérant de manière analogue à celle décrite dans les exemples 1 et 2 ou 3 et 4, en utilisant au départ le chlorure d'α-(acétylthiométhyl)benzène propanoyle et une amine appropriée, puis en saponifiant le produit obtenu, on a préparé les produits dont les noms suivent:

**Exemple 5**:

Ethanethioate de S-/3-(3,4-diméthoxyphényl)amino) 3-oxo 2-(phénylméthyl)propyle/

**Exemple 6**: N-(3,4-diméthoxyphényl) α-mercaptométhyl

benzène propanamide.

**Exemple 7**: Ethanethioate de S-/3-(2-benzothiazolylamino)3-oxo

2-(phénylméthyl) propyle/

**Exemple 8**: N-(2-benzothiazolyl) α-(mercaptométhyl)benzène

propanamide.

**Exemple 9**:

Ethanethioate de S-/3-(2-aminophénylamino)3-oxo 2-(phénylméthyl) propyle/

**Exemple 10**:

N-(2-aminophényl) α-(mercaptométhyl)benzène propanamide.

**Exemple 11**:

Ethanethioate de S-/3-(3-aminophénylamino)3-oxo 2(-phénylméthyl) propyle/

**Exemple 12**:

N-(3-aminophényl) α-(mercaptométhyl)benzène propanamide

**Exemple 13**:

Ethanethioate de S- /3-(4-aminophénylamino) 3-oxo 2-(phénylméthyl) propyle/

**Exemple 14**:

N-(4-aminophényl) α-(mercaptométhyl)benzène propanamide.

**Exemple 15**:

Ethanethioate de S-/3-(3-diméthylaminophényl amino)3-oxo 2-(phénylméthyl)propyle/

**Exemple 16**:

N-(3-diméthylaminophényl) α-(mercaptométhyl) benzène propanamide.

**Exemple 17**: Ethanethioate de S-/3-(4-diméthylaminophényl amino)3-oxo 2-(phénylméthyl)propyle/

**Exemple 18**:

N-(4-diméthylaminophényl) α-(mercaptométhyl) benzène propanamide.

**Exemple 19**:

Ethanethioate de S- /3-(2-hydroxyphénylamino) 3-oxo 2-(phénylméthyl)propyle/

**Exemple 20**:

N-(2-hydroxyphényl) α-(mercaptométhyl)benzène propanamide.

**Exemple 21**:

Ethanethioate de S-/3-(2,6-diméthylphénylamino) 3-oxo 2-(phénylméthyl)propyle/

**Exemple 22**:

N-(2,6-diméthylphényl) α-(mercaptométhyl)benzène propanamide.

**Exemple 23**:

Ethanethioate de S-/3-(4-hydroxyphénylamino)3-oxo 2-(phénylméthyl)propyle/

**Exemple 24**:

N-(4-hydroxyphényl) α-(mercaptométhyl)benzène propanamide.

**Exemple 25**:

Ethanethioate de S-/3-(3-hydroxyphénylamino)3-oxo 2-(phénylméthyl)propyle/

**Exemple 26**:

N-(3-hydroxyphényl) α-(mercaptométhyl)benzène propanamide.

**Exemple 27**:

Ethanethioate de S-/3-(2-chlorophénylamino)3-oxo 2-(phénylméthyl)propyle/

**Exemple 28**:

N-(2-chlorophényl) α-(mercaptométhyl)benzène propanamide.

**Exemple 29**:

Ethanethioate de S-/3-(4-pyridinylamino)3-oxo 2-(phénylméthyl)propyle/

**Exemple 30**:

N-(4-pyridinyl) α-(mercaptométhyl)benzène propanamide et son chlorhydrate.

**Exemple 31**:

Ethanethioate de S-/3-(3-chlorophénylamino)3-oxo 2-(phénylméthyl)propyle/

**Exemple 32**:

N-3-(chlorophényl) α-(mercaptométhyl)benzène propanamide.

**Exemple 33**:

Ethanethioate de S-/3-(2-thiazolylamino)3-oxo 2-(phénylméthyl)propyle.

**Exemple 34**:

N-(2-thiazolyl) α-(mercaptométhyl)benzène propanamide.

**Exemple 35**:

Ethanethioate de S-/3-(4-méthoxyphénylamino)3-oxo 2-(phénylméthyl)propyle.

**Exemple 36**:

N-(4-méthoxyphényl) α-(mercaptométhyl)benzène propanamide.

**Exemple 37**:

Ethanethioate de S-/3-(3-méthoxyphénylamino)3-oxo 2-(phénylméthyl)propyle.

**Exemple 38**:

N-(3-méthoxyphényl) α-(mercaptométhyl)benzène propanamide.

**Exemple 39**:

Ethanethioate de S-/3-(2-méthoxyphénylamino)3-oxo 2-(phénylméthyl) propyle.

**Exemple 40**:

N-(2-méthoxyphényl) α-(mercaptométhyl)benzène propanamide.

**Exemple 41**:

Ethanethioate de S-/3-(3-pyridinylamino)3-oxo 2-(phénylméthyl)propyle/

**Exemple 42**:

N-(3-pyridinyl) α-(mercaptométhyl)benzène propanamide et son méthane sulfonate.

**Exemple 43**:

Ethanethioate de S-/3-(4-méthoxycarbonylméthyl phénylamino)3-oxo 2-(phénylméthyl)propyle/

**Exemple 44**:

N-(4-méthoxycarbonylméthylphényl) α-(mercapto méthyl)benzène propanamide.

**Exemple 45**:

Ethanethioate de S-/3-1H-benzimidazol-2-ylamino) 3-oxo 2-(phénylméthyl)propyle/

**Exemple 46**:

N-(1H-benzimidazol-2-yl) α-(mercaptométhyl) benzène propanamide, méthane sulfonate.

**Exemple 47**:

Ethanethioate de S-/3-(4,5-dihydro 2-thiazolyl amino)3-oxo 2-(phénylméthyl)propyle/

**Exemple 48**:

Ethanethioate de S-/3-(3-phénylméthyloxyphényl amino)3-oxo propyle/

**Exemple 49**:

N-(3-phénylméthyloxyphényl)1-(mercaptométhyl) acétamide.

**Exemple 50**:

Ethanethioate de S-/3-phénylamino 3-oxo 2-méthyl propyle/

**Exemple 51**:

N-phényl 2-(mercaptométhyl)propanamide.

**Exemple 52**:

Ethanethioate de S-/3-amino 3-oxo 2-(phénylméthyl) benzène propanamide.

**Exemple 53**:

α-(mercaptométhyl)benzène propanamide.

La nature des substituants, les principales conditions opératoires et les constantes physiques des produits des exemples 5 à 53 sont reportées dans les tableaux ci-après:

| Exemples | $R_1$ | $R_2$ | $R_3$ | Solvant/ Saponification | Temp. en °C | Constantes |
|---|---|---|---|---|---|---|
| 5 | $CH_3CO$ | $-CH_2-\phi$ | 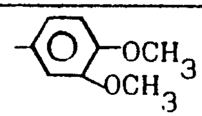 | $CH_2Cl_2$ | 0°C | F = 102°C |
| 6 | H | $-CH_2-\phi$ | ″ | idem Ex. 2 | 0°C | F = 131°C |
| 7 | $CH_3CO$ | $-CH_2-\phi$ | 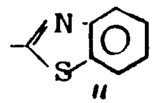 | $CH_2Cl_2$ | 0°C | F ≃ 50°C |
| 8 | H | $-CH_2-\phi$ | ″ | idem Ex. 2 | 0°C | F = 149°C |
| 9 | $CH_3CO$ | $-CH_2-\phi$ | 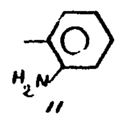 | $CH_2Cl_2$ | 0°C | F = 119°C |
| 10 | H | $-CH_2-\phi$ | ″ | idem Ex. 2 | 0°C | F = 136°C |
| 11 | $CH_3CO$ | $-CH_2-\phi$ | 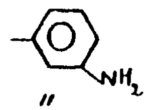 | $CH_2Cl_2$ | 0°C | F = 97°C |
| 12 | H | $-CH_2-\phi$ | ″ | idem Ex. 2 | 0°C | F = 122°C |
| 13 | $CH_3CO$ | $-CH_2-\phi$ | 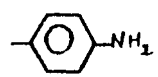 | $CH_2Cl_2$ | -10°C | F = 128°C |
| 14 | H | $-CH_2-\phi$ | ″ | idem Ex. 2 | - 5°C | F = 138°C |
| 15 | $CH_3CO$ | $-CH_2-\phi$ | 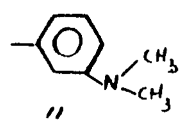 | $CH_2Cl_2$ | 0°C | F = 120°C |
| 16 | H | $-CH_2-\phi$ | ″ | idem Ex. 2 | 0°C | F = 98°C |
| 17 | $CH_3CO$ | $-CH_2-\phi$ | 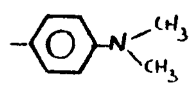 | $CH_2Cl_2$ | 0°C | F = 115°C |
| 18 | H | $-CH_2-\phi$ | ″ | idem Ex. 2 | 0°C | F = 124°C |
| 19 | $CH_3CO$ | $-CH_2-\phi$ | 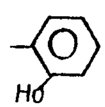 | $CH_3COC_2H_5$ | - 5°C | F = 88°C |
| 20 | H | $-CH_2-\phi$ | ″ | idem Ex. 2 | 0°C | F = 84°C |

| Exemples | $R_1$ | $R_2$ | $R_3$ | Solvant / Saponification | Temp. en °C | Constantes |
|---|---|---|---|---|---|---|
| 21 | $CH_3CO$ | $-CH_2-\phi$ | 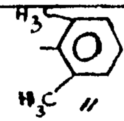 | $CH_2Cl_2$ | 0°C | F = 120°C |
| 22 | H | $-CH_2-\phi$ | " | idem Ex. 2 | 0°C | F = 160°C |
| 23 | $CH_3CO$ | $-CH_2-\phi$ | 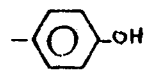 | $CH_3COC_2H_5$ | - 5°C | F = 120°C |
| 24 | H | $-CH_2-\phi$ | " | idem Ex. 2 | 0°C | F ≃ 50°C |
| 25 | $CH_3CO$ | $-CH_2-\phi$ | 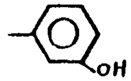 | $CHCl_3$ | 0°C | F = 102°C |
| 26 | H | $-CH_2-\phi$ | " | idem Ex. 2 | 0°C | F = 167-168°C |
| 27 | $CH_3CO$ | $-CH_2-\phi$ | 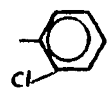 | $CH_2Cl_2$ | 0°C | F = 100°C |
| 28 | H | $-CH_2-\phi$ | " | idem Ex. 2 | 0°C | F = 83°C |
| 29 | $CH_3CO$ | $-CH_2-\phi$ | 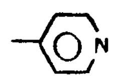 | THF | 0°C | F < 50°C |
| 30 | H | $-CH_2-\phi$ | ", HCl | idem Ex. 2 $Et_2O/HCl$ | 0°C | F = 167°C |
| 31 | $CH_3CO$ | $-CH_2-\phi$ | 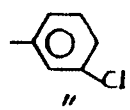 | $CH_2Cl_2$ | 0°C | F = 113°C |
| 32 | H | $-CH_2-\phi$ | " | idem Ex. 2 | 0°C | F = 121°C |
| 33 | $CH_3CO$ | $-CH_2-\phi$ | 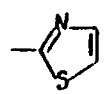 | $CH_2Cl_2$ | 0°C | F = 103°C |
| 34 | H | $-CH_2-\phi$ | " | idem Ex. 2 | 0°C | F = 90°C |
| 35 | $CH_3CO$ | $-CH_2-\phi$ | 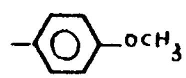 | $CH_2Cl_2$ | 0°C | F = 118°C |
| 36 | H | $-CH_2-\phi$ | " | idem Ex. 2 | 0°C | F = 115°C |
| 37 | $CH_3CO$ | $-CH_2-\phi$ | 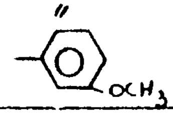 | $CH_2Cl_2$ | 0 C | F = 115°C |

| Exemples | $R_1$ | $R_2$ | $R_3$ | Solvant/ Saponification | Temp. en °C | Constantes |
|---|---|---|---|---|---|---|
| 38 | H | $-CH_2-\phi$ | 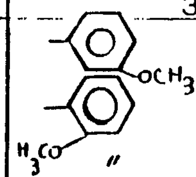 | idem Ex. 2 | 0°C | F = 72-73°C |
| 39 | $CH_3CO$ | $-CH_2-\phi$ | | $CH_2Cl_2$ | 0°C | - |
| 40 | H | $-CH_2-\phi$ | 〃 | idem Ex. 2 | - 5°C | F ≃ 50°C |
| 41 | $CH_3CO$ | $-CH_2-\phi$ | 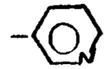 | $CH_2Cl_2$ | 0°C | F = 124-125°C |
| 42 | H | $-CH_2-\phi$ | 〃 et son sel de $CH_3SO_3H$ | idem Ex. 2<br>$CH_3SO_3H/Et_2O$ | 0°C<br>0,-5°C | F = 103-104°C<br>F = 146°C |
| 43 | $CH_3CO$ | $-CH_2-\phi$ | 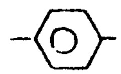 $CH_2-CO_2CH_3$ | $CH_2Cl_2$+ 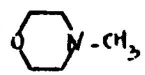 | -10°C | - |
| 44 | H | $-CH_2-\phi$ | 〃 | idem Ex. 2 | 0°C | F = 98°C |
| 45 | $CH_3CO$ | $-CH_2-\phi$ | 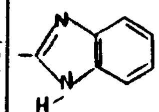 | THF | 0°C | F = 70-75°C |
| 46 | H | $-CH_2-\phi$ | 〃 , $CH_3SO_3H$ | idem Ex.2<br>$CH_3SO_3H/Et_2O$ | 0°C<br>- 9°C | F = 142°C |
| 47 | $CH_3CO$ | $-CH_2-\phi$ | 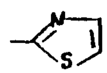 | $CH_2Cl_2$ | -10°C | F = 114°C |
| 48 | $CH_3CO$ | H | 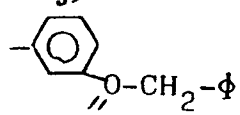 | $CH_2Cl_2$ | - 7°C | F = +87°C |
| 49 | H | H | 〃 | idem Ex. 2 | -10°C | F = +99°C |
| 50 | $CH_3CO$ | $CH_3$ | 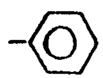 | $CH_2Cl_2$ | -10°C | F = 106°C |
| 51 | H | $CH_3$ | 〃 | idem Ex. 2 | 0°C | F = 100°C |
| 52 | $CH_3CO$ | $-CH_2-\phi$ | H | $CH_2Cl_2$ | 0°C | F = 78°C |
| 53 | H | $-CH_2-\phi$ | H | idem Ex. 2 | 0°C | F = 69°C |

Préparation du composé de départ de l'exemple 43:

**Chlorhydrate de (4-aminophényl) acétate de méthyle.**

On mélange 25,26 g d'acide (4-aminophényl)acétique et 200 $cm^3$ de méthanol. On fait barboter de l'acide chlorhydrique gazeux. On porte au reflux pendant une heure puis laisse revenir à 20° C. On évapore le solvant à 45° C, triture le résidu dans l'éther et le sèche. On obtient 32,2 g de produit attendu. F ≅165° C.

**Exemple 54**:

Compositions pharmaceutiques

On a préparé des comprimés répondant à la formule:

| | |
|---|---|
| Composé de l'exemple 1 | 50 mg |
| Excipient q.s.pour un comprimé terminé à | 350 mg |

(Détail de l'excipient: lactose, amidon, stéarate de magnésium, talc).

**Exemple 55**:

On a préparé des comprimés répondant à la formule:

| | |
|---|---|
| Composé de l'exemple 2 | 200 mg |
| Excipient pour un comprimé terminé à | 350 mg |

(Détail de l'excipient: lactose, amidon, stéarate de magnésium, talc).

**Exemple 56**:

On a préparé des comprimés répondant à la formule:

| | |
|---|---|
| Composé de l'exemple 38 | 50 mg |
| Excipient pour un comprimé terminé à | 350 mg |

(Détail de l'excipient: lactose, amidon, stéarate de magnésium, talc).

**Etude biologique**

1.- Dosage de l'enképhalinase et détermination de l'effet des inhibiteurs.

L'activité de l'enképhalinase est déterminée dans une fraction membranaire de striatum de rat.

Les striatum sont prélevés sur glace et homogénéisés en tampon TRIS 0.05M pH 7,4 (20 fois le volume). Après une première centrifugation à 1000 g, la fraction particulaire est obtenue à la suite de deux centrifugations de 10 minutes à 20 000 g. Le culot est ensuite mis en suspension dans un tampon TRIS et conservé à 4° C. Le dosage des protéines est effectué selon la méthode au bleu de Comassie.

Après une préincubation de 15 minutes à 25° C, une aliquote de protéines est incubée pendant 15 minutes à 25° C en présence de 20 nanomoles de leucine-enképhaline tritiée (sur le premier acide aminé) préalablement purifiée d'1 mM de puromycine et du produit à tester en tampon TRIS. La réaction d'hydrolyse est stoppée par addition d'acide chlorhydrique 0,2N et l'incubat subit une déprotéinisation par la chaleur (15 minutes à 95° C). Dans ces conditions, la cinétique de la réaction est linéaire.

Les métabolites tritiés obtenus par hydrolyse sont séparés de l'enképhaline par chromatographie sur colonne de porapak Q: ils sont élués au tampon TRIS tandis que l'enképhaline est retenue sur la colonne et recueillie ensuite en phase éthanolique.

L'activité des différents produits est exprimée en concentrations inhibitrices 50%.

**Résultats**:

| Produit de l'exemple | Inhibition de l'enképhalinase CI 50 en $10^{-6}$ M |
|---|---|
| 1 | 10 |
| 2 | 1 |
| 3 | 1 |

| | |
|---|---|
| 4 | 1 |
| 30 | 0,1 |
| 36 | 0,05 |
| 38 | 0,06 |

2. - <u>Test d'analgésie sur tissus inflammés</u>
(variante de la technique de Randall et Selitto (1)).

La technique consiste à rechercher l'effet analgésique chez le rat dont le seuil de sensibilité à la douleur a été abaissé par une inflammation.

Cette dernière est obtenue par injection de carraghénine (0,25 mg/patte) sous l'aponévrose plantaire d'une patte postérieure. La douleur est provoquée par pression mécanique appliquée sur la face dorsale de la patte et augmentée régulièrement au moyen d'un analgésimètre.

Le seuil douloureux est apprécié par la pression nécessaire pour déclencher une réaction de retrait de la patte ou une réaction de vocalisation de l'animal.

Les produits sont administrés par voie buccale 4 heures après l'injection de l'irritant et les mesures du seuil douloureux sont effectuées immédiatement avant l'injection de l'irritant puis une heure après le traitement.

(1) Randall, L.O. and Selitto, J.J. "a method for measurement of analgesic on inflammed tissue. Arch. Int. Pharmacodyn, 1957, 111, 409.

**Résultats**:

| Produit de l'exemple | DA en mg/kg per os |
|---|---|
| 1 | 20 |
| 2 | 4 |
| 4 | 50 |
| 30 | 4 <DA <20 |

**Revendications**

pour les Etats contractants BE CH DE FR GB IT LI LU NL SE

1. Les composés de formule (I):

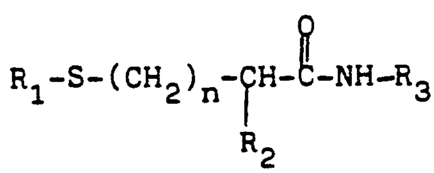

(I)

dans laquelle $R_1$ représente un atome d'hydrogène ou un radical

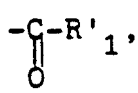

$R'_1$ étant un radical alcoyle renfermant de 1 à 5 atomes de carbone ou un radical aryle éventuellement substitué par un radical hydroxy, un radical alcoyle renfermant de 1 à 5 atomes de carbone, un radical alcoxy renfermant de 1 à 5 atomes de carbone, le radical nitro ou un atome d'halogène, n représente un nombre entier pouvant varier de 1 à 5, $R_2$ représente un atome d'hydrogène, un radical alcoyle renfermant de 1 à 5 atomes de carbone, un radical aryle ou arylalcoyle renfermant de 6 à 15 atomes de carbone, éventuellement substitués par un radical alcoyle renfermant de 1 à 5 atomes de carbone, un radical alcoxy renfermant de 1 à 5 atomes de carbone, un radical hydroxyle, un atome d'halogène ou un radical trifluorométhyle, $R_3$ représente un radical hétérocyclique choisi parmi les radicaux thiazolyle, 4,5-dihydrothiazolyle, pyridinyle, oxazolyle, isoxazolyle, imidazolyle, pyrimidyle, tétrazolyle benzimidazolyle, benzothiazolyle ou benzoxazolyle, éventuellement substitué par un radical alcoyle renfermant de 1 à 5 atomes de carbone ou $R_3$ représente un atome d'hydrogène, un radical phényle éventuellement substitué par un ou plusieurs radicaux choisis dans le groupe constitué par les radicaux alcoyles renfermant de 1 à 5 atomes de carbone, les radicaux alcoxy renfermant de 1 à 5 atomes de carbone, le radical hydroxyle, le radical nitro, les atomes d'halogènes, le radical

trifluorométhyle, le radical carboxyméthyle, les radicaux alcoxy carbonylméthyle dans lesquels le radical alcoxy renferme de 1 à 5 atomes de carbone, les radicaux aralcoyloxy renfermant de 7 à 15 atomes de carbone et les radicaux:

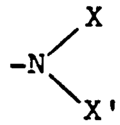 , ;

dans lesquels X et X', identiques ou differents, représentent un atome d'hydrogène ou un radical alcoyle renfermant de 1 à 5 atomes de carbone, $R_3$ ne pouvant toutefois représenter un radical phényle non substitué ou substitué par un ou plusieurs radicaux choisis dans le groupe constitué par les radicaux alcoyles renfermant de 1 à 5 atomes de carbone, les radicaux alcoxy renfermant de 1 à 5 atomes de carbone, et les atomes d'halogènes, lorsque $R_2$ représente un atome d'hydrogène et lorsque n est égal 1 ou à 2 et $R_3$ ne pouvant représenter un radical phényle substitué par un radical carboxyméthyle lorsque $R_2$ représente un radical méthyle et lorsque n est égal à 1, ainsi que leurs sels d'addition avec les acides et les bases.

2. Les composés de formule (I) tels que définis à la revendication 1 pour lesquels $R_1$ est un atome d'hydrogène ainsi que leurs sels d'addition avec les acides et les bases.

3. Les composés de formule (I) tels que définis à la revendication 1, pour lesquels $R_1$ est un radical acétyle, ainsi que leurs sels d'addition avec les acides et les bases.

4. Les composés de formule (I) tels que définis à l'une quelconque des revendications 1 à 3, pour lesquels $R_2$ ne représente pas un atome d'hydrogène, ainsi que leurs sels d'addition avec les acides et les bases.

5. Les composés de formule (I) tels que définis à l'une quelconque des revendications 1 à 4, pour lesquels n = 1 et $R_2$ est un radical benzyle, ainsi que leurs sels d'addition avec les acides et les bases.

6. Les composés de formule (I) tels que définis à l'une quelconque des revendications 1 à 5, pour lesquels $R_3$ est un radical phényle éventuellement substitué par un ou plusieurs atomes de chlore ou par un ou plusieurs radicaux méthoxy ou un radical pyridinyle, ainsi que leurs sels d'addition avec les acides et les bases.

7. L'un quelconque des composés de formule (I) selon la revendication 1 dont les noms suivent:
- l'α-(mercaptométhyl)N-phényl benzène propanamide,
- l'éthanethioate de S-/3-oxo-3-(phénylamino)2-(phénylméthyl)propyl/
- l'α-(mercaptométhyl)N-(3-méthoxyphényl)benzène propanamide,
- l'α-(mercaptométhyl)N-(4-méthoxyphényl)benzène propanamide,
- l'α-(mercaptométhyl)N-(4-pyridinyl)benzène propanamide,
ainsi que leurs sels d'addition avec les acides et les bases.

8. Procédé de préparation des produits de formule (I) dans laquelle $R_1$, $R_2$, et $R_3$ ont les significations données précédemment et dans laquelle n = 1, caractérisé en ce que l'on soumet un acide de formule (II):

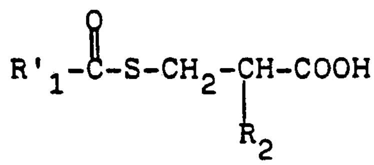 (II)

dans laquelle $R'_1$ et $R_2$ ont les significations déjà indiquées ou un dérivé fonctionnel de cet acide, à l'action d'un produit de formule (III):

$$H_2N - R_3 \quad (III)$$

dans laquelle $R_3$ a la signification déjà indiquée, pour obtenir un produit de formule (I) dans laquelle:

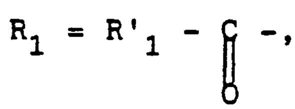

$R'_1$ ainsi que $R_2$ et $R_3$ ont les significations déjà indiquées et dans laquelle n = 1, produit de formule (I) que, le cas échéant, l'on saponifie, pour obtenir un produit de formule (I) dans laquelle $R_1$ représente un atome d'hydrogène, produits de formule (I) que l'on soumet, si désiré, à l'action d'une base ou d'un acide pour en former le sel.

9. Procédé de préparation des produits de formule (I°) dans laquelle $R_1$, $R_2$ et $R_3$ ont les significations données précédemment, et dans laquelle n représente un nombre entier pouvant varier et de 1 à 5, caractérisé en ce que l'on soumet un acide de formule (IV):

$$X_1-(CH_2)_n-\overset{\displaystyle R_2}{\overset{|}{C}}H-COOH \qquad (IV)$$

dans laquelle $X_1$ est un atome d'halogène et dans laquelle n et $R_2$ ont les significations données ci-dessus ou un dérivé fonctionnel de celui-ci, à l'action d'un produit de formule (III) ($NH_2$-$R_3$) précédent, pour obtenir un produit de formule (V):

$$X_1-(CH_2)_n-\overset{\displaystyle R_2}{\overset{|}{C}}H-CONHR_3 \qquad (V)$$

dans laquelle $X_1$, n, $R_2$ et $R_3$ ont les significations précédentes, que l'on soumet à l'action de l'anion d'un thioacide de formule VI:

$$R'_1-\underset{\displaystyle O}{\underset{\|}{C}}-SH \qquad (VI)$$

dans laquelle $R'_1$ conserve sa signification, pour obtenir un produit de formule (I) dans laquelle $R_1$ =

$$R'_1-\underset{\displaystyle O}{\underset{\|}{C}}-,$$

n représente un nombre entier pouvant varier de 1 à 5 et dans laquelle $R'_1$, $R_2$ et $R_3$ ont les significations données ci-dessus, produit de formule (I) que, le cas échéant, l'on saponifie, pour obtenir un produit de formule (I) dans laquelle $R_1$ représente un atome d'hydrogène, produits de formule (I) que l'on soumet, si désiré, à l'action d'un acide ou d'une base pour en former le sel.

10. A titre de médicaments, les produits de formule (I) tels que définis à l'une quelconque des revendications 1 à 6, ainsi que leurs sels d'addition avec les acides ou les bases pharmaceutiquement acceptables.

11. A titre de médicaments, les produits tels que définis par la revendication 7, ainsi que leurs sels d'addition avec les acides et les bases pharmaceutiquement acceptables.

12. Les compositions pharmaceutiques contenant à titre de principe actif, l'un au moins des médicaments tels que définis par la revendication 10 ou 11.

13. A titre de produits industriels nouveaux, les produits de formule (V) tels que définis à la revendication 9.

**Revendications**

pour l'Etat contractant AT

1. Procédé pour préparer les composés de formule (I):

$$R_1-S-(CH_2)_n-\underset{\displaystyle R_2}{\underset{|}{C}}H-\overset{\displaystyle O}{\overset{\|}{C}}-NH-R_3 \qquad (I)$$

dans laquelle $R_1$ représente un atome d'hydrogène ou un radical

$$-\underset{\displaystyle O}{\underset{\|}{C}}-R'_1,$$

$R'_1$ étant un radical alcoyle renfermant de 1 à 5 atomes de carbone ou un radical aryle éventuellement substitué par un radical hydroxy, un radical alcoyle renfermant de 1 à 5 atomes de carbone, un radical alcoxy renfermant de 1 à 5 atomes de carbone, le radical nitro ou un atome d'halogène, n représente un nombre entier pouvant varier de 1 à 5, $R_2$ représente un atome d'hydrogène, un radical alcoyle renfermant de 1 à 5 atomes de carbone, un radical aryle ou arylalcoyle renfermant de 6 à 15 atomes de carbone, éventuellement substitués par un radical alcoyle renfermant de 1 à 5 atomes de carbone, un radical alcoxy renfermant de 1 à 5 atomes de carbone, un radical hydroxyle, un atome d'halogène ou un radical trifluorométhyle, $R_3$ représente un radical

hétérocyclique choisi parmi les radicaux thiazolyle, 4,5-dihydrothiazolyle, pyridinyle, oxazolyle, isoxazolyle, imidazolyle, pyrimidyle, tétrazolyle benzimidazolyle, benzothiazolyle ou benzoxazolyle, éventuellement substitué par un radical alcoyle renfermant de 1 à 5 atomes de carbone ou $R_3$ représente un atome d'hydrogène, un radical phényle éventuellement substitué par un ou plusieurs radicaux choisis dans le groupe constitué par les radicaux alcoyles renfermant de 1 à 5 atomes de carbone, les radicaux alcoxy renfermant de I à 5 atomes de carbone, le radical hydroxyle, le radical nitro, les atomes d'halogènes, le radical trifluorométhyle, le radical carboxyméthyle, les radicaux alcoxy carbonylméthyle dans lesquels le radical alcoxy renferme de 1 à 5 atomes de carbone, les radicaux aralcoyloxy renfermant de 7 à 15 atomes de carbone et les radicaux:

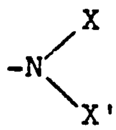 ,

dans lesquels X et X', identiques ou différents, représentent un atome d'hydrogène ou un radical alcoyle renfermant de 1 à 5 atomes de carbone, $R_3$ ne pouvant toutefois représenter un radical phényle non substitué ou substitué par un ou plusieurs radicaux choisis dans le groupe constitué par les radicaux alcoyles renfermant de 1 à 5 atomes de carbone, les radicaux alcoxy renfermant de 1 à 5 atomes de carbone, et les atomes d'halogènes, lorsque $R_2$ représente un atome d'hydrogène et lorsque n est égal à 1 ou à 2 et $R_3$ ne pouvant représenter un radical phényle substitué par un radical carboxyméthyle lorsque $R_2$ représente un radical méthyle et lorsque n est égal à 1, ainsi que leurs sels d'addition avec les acides et les bases, caractérisé en ce que:

- pour préparer les composés de formule (I) dans laquelle $R_1$, $R_2$ et $R_3$ ont la signification précitée et dans laquelle n = 1, ainsi que leurs sels d'addition avec les acides et les bases, l'on soumet un acide de formule (II):

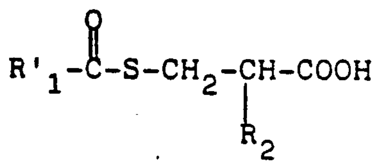 (II)

dans laquelle $R'_1$ et $R_2$ ont les significations déjà indiquées ou un dérivé fonctionnel de cet acide, à l'action d'un produit de formule (III):

$$H_2N - R_3 \qquad (III)$$

dans laquelle $R_3$ a la signification déjà indiquée, pour obtenir un produit de formule (I) dans laquelle: $R_1 = R'_1$

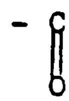

-, $R'_1$ ainsi que $R_2$ et $R_3$ ont les significations déjà indiquées et dans laquelle n = 1, produit de formule (I) que, le cas échéant, l'on saponifie, pour obtenir un produit de formule (I) dans laquelle $R_1$ représente un atome d'hydrogène, produits de formule (I) que l'on soumet, si désiré, à l'action d'une base ou d'un acide pour en former le sel;

- pour préparer les composés de formule (I) dans laquelle $R_1$, $R_2$ et $R_3$ ont la signification précitée et dans laquelle n est un nombre entier variant de 1 à 5, ainsi que leurs sels d'addition avec les acides et les bases, l'on soumet un acide de formule (IV):

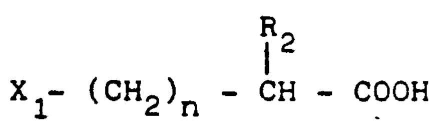 (IV)

dans laquelle $X_1$ est un atome d'halogène et dans laquelle n et $R_2$ ont les significations données ci-dessus ou un dérivé fonctionnel de celui-ci, à l'action d'un produit de formule (III) ($NH_2$-$R_3$) précédent, pour obtenir un produit de formule (V):

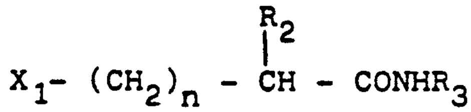

(V)

dans laquelle $X_1$ n, $R_2$ et $R_3$ ont les significations précédentes, que l'on soumet à l'action de l'anion d'un thioacide de formule VI:

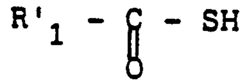

(VI)

dans laquelle $R'_1$ conserve sa signification, pour obtenir un produit de formule (I) dans laquelle $R_1$ =

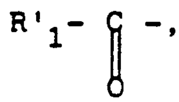

n représente un nombre entier pouvant varier de 1 à 5 et dans laquelle $R'_1$, $R_2$ et $R_3$ ont les significations données ci-dessus, produit de formule (I) que, le cas échéant, l'on saponifie, pour obtenir un produit de formule (I) dans laquelle $R_1$ représente un atome d'hydrogène, produits de formule (I) que l'on soumet, si désiré, à l'action d'un acide ou d'une base pour en former le sel.

2. Procédé selon la revendication 1, pour la préparation des composés de formule (I) dans laquelle $R_1$, $R_2$ et $R_3$ ont la signification précitée et dans laquelle n = 1, ainsi que leurs sels d'addition avec les acides et les bases, caractérisé en ce que l'on soumet un acide de formule (II):

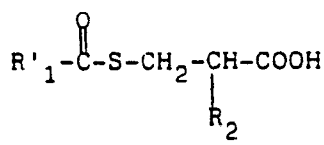

(II)

dans laquelle $R'_1$ et $R_2$ ont les significations déjà indiquées ou un dérivé fonctionnel de cet acide, à l'action d'un produit de formule (III):

$$H_2N - R_3 \qquad (III)$$

dans laquelle $R_3$ a la signification déjà indiquée, pour obtenir un produit de formule (I) dans laquelle: $R_1$ =

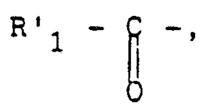

$R'_1$ ainsi que $R_2$ et $R_3$ ont les significations déjà indiquées et dans laquelle n = 1,

produit de formule (I) que, le cas échéant, l'on saponifie, pour obtenir un produit de formule (I) dans laquelle $R_1$ représente un atome d'hydrogène, produits de formule (I) que l'on soumet, si désiré, à l'action d'une base ou d'un acide pour en former le sel.

3. Procédé selon la revendication 1, pour la préparation des composés de formule (I), dans laquelle $R_1$, $R_2$ et $R_3$ ont la signification précitée et dans laquelle n est un nombre entier variant de 1 à 5, ainsi que leurs sels d'addition avec les acides et les bases, caractérisé en ce que l'on soumet un acide de formule (IV):

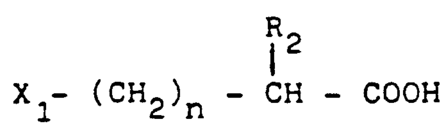

(IV)

dans laquelle $X_1$ est un atome d'halogène et dans laquelle n et $R_2$ ont les significations données ci-dessus ou un dérivé fonctionnel de celui-ci, à l'action d'un produit de formule (III) ($NH_2$-$R_3$) précédent, pour obtenir un produit de formule (V):

$$X_1-(CH_2)_n-\underset{|}{\overset{R_2}{CH}}-CONHR_3 \qquad (V)$$

dans laquelle $X_1$ n, $R_2$ et $R_3$ ont les significations précédentes, que l'on soumet à l'action de l'anion d'un thioacide de formule VI:

$$R'_1-\underset{\underset{O}{\|}}{C}-SH \qquad (VI)$$

dans lacuelle $R'_1$ conserve sa signification, pour obtenir un produit de formule (I) dans laquelle R =

$$R'_1-\underset{\underset{O}{\|}}{C}-,$$

n représente un nombre entier pouvant varier de 1 à 5 et dans laquelle $R'_1$, $R_2$ et $R_3$ ont les significations données ci-dessus, produit de formule (I) que, le cas échéant, l'on saponifie, pour obtenir un produit de formule (I) dans laquelle $R_1$ représente un atome d'hydrogène, produits de formule (I) que l'on soumet, si désiré, à l'action d'un acide ou d'une base pour en former le sel.

4. Procédé selon la revendication 1, pour la préparation des composés de formule (I) telle que définie à la revendication 1, répondant à la formule (I'):

$$R_1-S-(CH_2)_n-\underset{\underset{R_2}{|}}{CH}-\overset{\overset{O}{\|}}{C}-NH\ R'_3 \qquad (I')$$

dans laquelle $R_1$, $R_2$ et n conservent leurs significations précitées et $R'_3$ représente un radical hétérocyclique choisi parmi les radicaux thiazolyle, 4,5-dihydrothiazolyle, pyridinyle, oxazolyle, isoxazolyle, imidazolyle, pyrimidyle, tétrazolyle, benzimidazolyle, éventuellement substitué par un radical alcoyle renfermant de 1 à 5 atomes de carbone ou $R_3$ représente un radical phényle éventuellement substitué par un ou plusieurs radicaux choisis dans le groupe constitué par les radicaux alcoyles renfermant de 1 à 5 atomes de carbone, les radicaux alcoxy renfermant de 1 à 5 atomes de carbone, le radical hydroxyle, le radical nitro, les atomes d'halogènes, le radical trifluorométhyle et les radicaux

$$-N{<}^{X}_{X'}$$

X et X' indentiques ou différents représentent un atome d'hydrogène ou un radical alcoyle renfermant de 1 à 5 atomes de carbone, $R_3$ ne pouvant toutefois représenter un radical phényle substitué ou non substitué, lorsque $R_2$ représente un atome d'hydrogène et lorsque n est égal à 1, ainsi que de leurs sels d'addition avec les acides et les bases, caractérisé en ce que l'on utilise au départ un produit de formule (III) dans laquelle $R_3$ a les valeurs de $R'_3$ indiquées ci-dessus.

5. Procédé selon la revendication 4, caractérisé en ce que l'on utilise au départ un produit de formule (II) ou (VI) dans laquelle

$$R'_1-\underset{\underset{O}{\|}}{C}-$$

représente un radical acétyle.

6.- Procédé selon la revendication 5, caractérisé en ce que l'on utilise au départ un produit de formule (II) ou (IV) dans laquelle $R_2$ représente un radical benzyle et n est égal à 1.

7. Procédé selon l'une quelconque des revendications 4 à 6, caractérisé en ce que l'on utilise au départ un produit de formule (III) dans laquelle $R_3$ est un radical phényle éventuellement substitué par un ou plusieurs atomes de chlore ou par un plusieurs radicaux méthoxy ou un radical pyridinyle.

8. Procedé selon l'une quelconque des revendications 1 à 4, caractérisé en ce que l'on prépare l'un quelconque des produits de formule (I) dont les noms suivent:

- l'α-(mercaptométhyl)N-phényl benzène propanamide,
- l'éthanethioate de S-/3- oxo-3-(phénylamino)2-(phénylméthyl)propyl/,

- l'α-(mercaptométhyl)N-(3-méthoxyphényl)benzène propanamide,
- l'α-(mercaptométhyl)N-(4-méthoxyphényl)benzène propanamide,
- l'α-(mercaptométhyl)N-(4-pyridinyl)benzène propanamide,
- ainsi que leurs sels d'addition avec les acides et les bases.

**Patentansprüche**

für die Vertragsstaaten: BE, CH, DE, FR, GB, IT, LI, LU, NL, SE

1. Verbindungen der Formel (I)

$$R_1\text{-}S\text{-}(CH_2)_n\text{-}\underset{R_2}{CH}\text{-}\overset{O}{\overset{\|}{C}}\text{-}NH\text{-}R_3 \qquad (I)$$

worin $R_1$ ein Wasserstoffatom oder einen Rest

$$\underset{O}{\underset{\|}{-C}}\text{-}R'_1$$

darstellt, wobei $R'_1$ einen Alkylrest mit 1 bis 5 Kohlenstoffatomen oder einen Arylrest, gegebenenfalls substituiert durch eine Hydroxygruppe, eine Alkylgruppe mit 1 bis 5 Kohlenstoffatomen, eine Alkoxygruppe mit 1 bis 5 Kohlenstoffatomen, die Nitrogruppe oder ein Halogenatom, bedeutet, n eine ganze Zahl von 1 bis 5 darstellt, $R_2$ ein Wasserstoffatom, einen Alkylrest mit 1 bis 5 Kohlenstoffatomen, einen Aryl- oder Aralkylrest mit 6 bis 15 Kohlenstoffatomen, gegebenenfalls substituiert durch einen Alkylrest mit 1 bis 5 Kohlenstoffatomen, einen Alkoxyrest mit 1 bis 5 Kohlenstoffatomen, einen Hydroxylrest, ein Halogenatom oder eine Trifluormethylgruppe bedeutet, $R_3$ einen heterocyclischen Rest, ausgewählt unter den Thiazolyl-, 4,5-Dihydrothiazolyl-, Pyridinyl-, Oxazolyl-, Isoxazolyl-, Imidazolyl-, Pyrimidyl-, Tetrazolyl-, Benzimidazolyl-, Benzothiazolyl- oder Benzoxazolylresten, gegebenenfalls substituiert durch einen Alkylrest mit 1 bis 5 Kohlenstoffatomen, bedeutet oder $R_3$ ein Wasserstoffatom, einen Phenylrest, der gegebenenfalls substituiert ist durch einen oder mehrere Reste, ausgewählt unter den Alkylresten mit 1 bis 5 Kohlenstoffatomen, den Alkoxyresten mit 1 bis 5 Kohlenstoffatomen, dem Hydroxylrest, der Nitrogruppe, den Halogenatomen, dem Trifluormethylrest, dem Carboxymethylrest, den Alkoxycarbonylmethylresten, worin die Alkoxygruppe 1 bis 5 Kohlenstoffatome umfaßt, den Aralkoxyresten mit 7 bis 15 Kohlenstoffatomen und den Resten

$$-N\begin{matrix}\diagup X\\ \diagdown X'\end{matrix},$$

worin X und X', die gleich oder voneinander verschieden sind, ein Wasserstoffatom oder einen Alkylrest mit 1 bis 5 Kohlenstoffatomen darstellen, bedeutet, wobei $R_3$ jedoch nicht einen unsubstituierten Phenylrest oder einen durch einen oder mehrere Reste, ausgewählt unter den Alkylresten mit 1 bis 5 Kohlenstoffatomen, den Alkoxyresten mit 1 bis 5 Kohlenstoffatomen und den Halogenatomen, substituierten Phenylrest bedeuten kann, wenn $R_2$ ein Wasserstoffatom darstellt und n für 1 oder 2 steht, und $R_3$ nicht einen durch einen Carboxymethylrest substituierten Phenylrest bedeuten kann, wenn $R_2$ einen Methylrest bedeutet und n für 1 steht, sowie deren Additionssalze mit Säuren und Basen.

2. Verbindungen der Formel (I) gemäß Anspruch 1, worin $R_1$ ein Wasserstoffatom darstellt, sowie deren Additionssalze mit Säuren und Basen.

3. Verbindungen der Formel (I) gemäß Anspruch 1, worin $R_1$ einen Acetylrest bedeutet, sowie deren Additionssalze mit Säuren und Basen.

4. Verbindungen der Formel (I) gemäß einem der Ansprüche 1 bis 3, worin $R_2$ kein Wasserstoffatom bedeutet, sowie deren Additionssalze mit Säuren und Basen.

5. Verbindungen der Formel (I) gemäß einem der Ansprüche 1 bis 4, worin n = 1 und $R_2$ einen Benzylrest bedeutet, sowie deren Additionssalze mit Säuren und Basen.

6. Verbindungen der Formel (I) gemäß einem der Ansprüche 1 bis 5, worin $R_3$ einen gegebenenfalls durch ein oder mehrere Chloratome oder einen oder mehrere Methoxyreste substituierten Phenylrest oder einen Pyridinylrest bedeutet, sowie deren Additionssalze mit Säuren und Basen.

7. Eine der Verbindungen der Formel (I) gemäß Anspuch 1 mit den folgenden Bezeichnungen:
α-(Mercaptomethyl)-N-phenylbenzolpropanamid,
S-[3-Oxo-3-)(phenylamino)-2-(phenylmethyl)-propyl]-ethanthioat,
α-(Mercaptomethyl)-N-(3-methoxyphenyl)-benzol-propanamid,

α-(Mercaptomethyl)-N-(4-methoxyphenyl)-benzol-propanamid,
α-(Mercaptomethyl)-N-(4-pyridinyl)-benzol-propanamid,
sowie deren Additionssalze mit Säuren und Basen.

8. Verfahren zur Herstellung der Produkte der Formel (I), worin $R_1$, $R_2$ und $R_3$ die vorstehend angegebene Bedeutung besitzen und worin n = 1, dadurch gekennzeichnet, daß man eine Säure der Formel (II)

$$R'_1-\overset{\overset{O}{\|}}{C}-S-CH_2-\underset{\underset{R_2}{|}}{CH}-COOH \qquad (II)$$

worin $R'_1$ und $R_2$ die angegebenen Bedeutungen besitzen, oder ein funktionelles Derivat dieser Säure der Einwirkung eines Produkts der Formel (III)

$$H_2N - R_3 \qquad (III)$$

worin $R_3$ die angegebene Bedeutung besitzt, unterzieht, um ein Produkt der Formel (I) zu erhalten, worin $R_1 =$ $R'_1$-

$$-\underset{\underset{O}{\|}}{C}-$$

$R'_1$ sowie $R_2$ und $R_3$ die angegebenen Bedeutungen besitzen und worin n = 1, das Produkt der Formel (I) gegebenenfalls verseift, um ein Produkt der Formel (I) zu erhalten, worin $R_1$ ein Wasserstoffatom bedeutet, die Produkte der Formel (I) gewünschtenfalls der Einwirkung einer Base oder einer Säure unterzieht, um hieraus das Salz zu bilden.

9. Verfahren zur Herstellung der Produkte der Formel (I), worin $R_1$, $R_2$ und $R_3$ die vorstehend angegebenen Bedeutungen besitzen und worin n eine ganze Zahl von 1 bis 5 darstellt, dadurch gekennzeichnet, daß man eine Säure der Formel (IV)

$$X_1 - (CH_2)_n - \overset{\overset{R_2}{|}}{CH} - COOH \qquad (IV)$$

worin X ein Halogenatom ist und worin n und $R_2$ die angegebenen Bedeutungen besitzen, oder ein funktionelles Derivat derselben der Einwirkung eines Produktes der vorstehenden Formel (III) $(NH_2\text{-}R_3)$ unterzieht, um ein Produkt der Formel (V)

$$X_1 - (CH_2)_n - \overset{\overset{R_2}{|}}{CH} - CONHR_3 \qquad (V)$$

zu erhalten, worin $X_1$, n, $R_2$ und $R_3$ die angegebene Bedeutung besitzen, das man der Einwirkung eines Anions einer Thiosäure der Formel (VI)

$$R'_1 - \underset{\underset{O}{\|}}{C} - SH \qquad (VI)$$

unterzieht, worin $R'_1$ die angegebene Bedeutung besitzt, um ein Produkt der Formel (I) zu erhalten, worin $R_1 =$

$$R'_1-\underset{\underset{O}{\|}}{C}- ,$$

n eine ganze Zahl von 1 bis 5 bedeutet und $R'_1$, $R_2$ und $R_3$ die vorstehend angegebenen Bedeutungen haben, das Produkt der Formel (I) gegebenenfalls verseift, um ein Produkt der Formel (I) zu erhalten, worin $R_3$ ein Wasserstoffatom bedeutet, und gewünschtenfalls die Produkte der Formel (I) der Einwirkung einer Säure oder einer Base unterzieht, um hieraus das Salz zu bilden.

10. Als Arzneimittel die Produkte der Formel (I) gemäß einem der Ansprüche 1 bis 6 sowie deren Additionssalze mit pharmazeutisch verträglichen Säuren oder Basen.

11. Als Arzneimittel die Produkte gemäß Anspruch 7 sowie deren Additionssalze mit pharmazeutisch verträglichen Säuren und Basen.

12. Pharmazeutische Zusammensetzungen, enthaltend als Wirkstoff zumindest eines der Arzneimittel gemäß Anspruch 10 oder 11.

13. Als neue, industrielle Produkte die Produkte der Formel (V) gemäß Anspruch 9.

**Patentansprüche**

für den Vertragsstaat: AT

1. Verfahren zur Herstellung der Verbindungen der Formel (I)

$$R_1\text{-}S\text{-}(CH_2)_n\text{-}\underset{\displaystyle R_2}{\underset{|}{CH}}\text{-}\overset{\displaystyle O}{\overset{\|}{C}}\text{-}NH\text{-}R_3 \qquad (I)$$

worin $R_1$ ein Wasserstoffatom oder einen Rest

$$-\underset{\displaystyle O}{\underset{\|}{C}}-R'_1$$

darstellt, wobei $R'_1$ einen Alkylrest mit 1 bis 5 Kohlenstoffatomen oder einen Arylrest, gegebenenfalls substituiert durch eine Hydroxygruppe, eine Alkylgruppe mit 1 bis 5 Kohlenstoffatomen, eine Alkoxygruppe mit 1 bis 5 Kohlenstoffatomen, die Nitrogruppe oder ein Halogenatom, bedeutet, n eine ganze Zahl von 1 bis 5 darstellt, $R_2$ ein Wasserstoffatom, einen Alkylrest mit 1 bis 5 Kohlenstoffatomen, einen Aryl- oder Aralkylrest mit 6 bis 15 Kohlenstoffatomen, gegebenenfalls substituiert durch einen Alkylrest mit 1 bis 5 Kohlenstoffatomen, einen Alkoxyrest mit 1 bis 5 Kohlenstoffatomen, einen Hydroxylrest, ein Halogenatom oder eine Trifluormethylgruppe, bedeutet, $R_3$ einen heterocyclischen Rest, ausgewählt unter den Thiazolyl-, 4,5-Dihydrothiazolyl-, Pyridinyl-, Oxazolyl-, Isoxazolyl-, Imidazolyl-, Pyrimidyl-, Tetrazolyl-, Benzimidazolyl-, Benzothiazolyl- oder Benzoxazolylresten, gegebenenfälls substituiert durch einen Alkylrest mit 1 bis 5 Kohlenstoffatomen, bedeutet oder $R_3$ ein Wasserstoffatom, einen Phenylrest, der gegebenenfalls substituiert ist durch einen oder mehrere Reste, ausgewählt unter den Alkylresten mit 1 bis 5 Kohlenstoffatomen, den Alkoxyresten mit 1 bis 5 Kohlenstoffatomen, dem Hydroxylrest, der Nitrogruppe, den Halogenatomen, dem Trifluormethylrest, dem Carboxymethylrest, den Alkoxycarbonylmethylresten, worin die Alkoxygruppe 1 bis 5 Kohlenstoffatome umfaßt, den Aralkoxyresten mit 7 bis 15 Kohlenstoffatomen und den Resten

$$-N{<}^{X}_{X'} \; ,$$

worin X und X', die gleich oder voneinander verschieden sind, ein Wasserstoffatom oder einen Alkylrest mit 1 bis 5 Kohlenstoffatomen darstellen, bedeutet, wobei $R_3$ jedoch nicht einen unsubstituierten Phenylrest oder einen durch einen oder mehrere Reste, ausgewählt unter den Alkylresten mit 1 bis 5 Kohlenstoffatomen, den Alkoxyresten mit 1 bis 5 Kohlenstoffatomen und den Halogenatomen, substituierten Phenylrest bedeuten kann, wenn $R_2$ ein Wasserstoffatom darstellt und n für 1 oder 2 steht, und $R_3$ nicht einen durch einen Carboxymethylrest substituierten Phenylrest bedeuten kann, wenn $R_2$ einen Methylrest bedeutet und n für 1 steht, sowie von deren Additionssalzen mit Säuren und Basen,

dadurch gekennzeichnet, daß

zur Herstellung der Verbindungen der Formel (I), worin $R_1$, $R_2$ und $R_3$ die angegebene Bedeutung besitzen und n = 1, sowie von deren Additionssalzen mit Säuren und Basen man eine Säure der Formel (II)

$$R'_1-\overset{\overset{O}{\parallel}}{C}-S-CH_2-\underset{\underset{R_2}{|}}{CH}-COOH \qquad (II)$$

worin $R'_1$ und $R_2$ die angegebenen Bedeutungen besitzen, oder ein funktionelles Derivat dieser Säure der Einwirkung eines Produktes der Formel (III)

$$H_2N - R_3 \qquad (III)$$

unterzieht, worin $R_3$ die angegebene Bedeutung besitzt, um ein Produkt der Formel (I) zu erhalten, worin $R_1$ = $R'_1$-

$$-\underset{\underset{O}{\parallel}}{C}-,$$

$R'_1$ sowie $R_2$ und $R_3$ die angegebenen Bedeutungen besitzen und worin n = 1, das Produkt der Formel (I) gegebenenfalls verseift, um ein Produkt der Formel (I) zu erhalten, worin $R_1$ ein Wasserstoffatom bedeutet, die Produkte der Formel (I) gewünschtenfalls der Einwirkung einer Base oder einer Säure unterzieht, um hieraus das Salz zu bilden;

zur Herstellung der Produkte der Formel (I), worin $R_1$, $R_2$ und $R_3$ die angegebene Bedeutung besitzen und worin n eine ganze Zahl von 1 bis 5 darstellt, sowie von deren Additionssalzen mit Säuren und Basen man eine Säure der Formel (IV)

$$X_1 - (CH_2)_n - \overset{\overset{R_2}{|}}{CH} - COOH \qquad (IV)$$

worin $X_1$ ein Halogenatom bedeutet und n und $R_2$ die vorstehend angegebenen Bedeutungen besitzen, oder ein funktionelles Derivat derselben der Einwirkung eines Produktes der vorstehenden Formel (III) ($NH_2$-$R_3$) unterzieht, um ein Produkt der Formel (V)

$$X_1 - (CH_2)_n - \overset{\overset{R_2}{|}}{CH} - CONHR_3 \qquad (V)$$

zu erhalten, worin $X_1$, n, $R_2$ und $R_3$ die vorstehenden Bedeutungen besitzen, das man der Einwirkung eines Anions einer Thiosäure der Formel VI

$$R'_1 - \underset{\underset{O}{\parallel}}{C} - SH \qquad (VI)$$

unterzieht, worin $R'_1$ dle angegebene Bedeutung besitzt, um ein Produkt der Formel (I) zu erhalten, worin $R_1$ = $R'_1$,

$$-\underset{\underset{O}{\parallel}}{C}-,$$

n eine ganze Zahl von 1 bis 5 darstellt und worin $R'_1$, $R_2$ und $R_3$ die vorstehend angegebenen Bedeutungen besitzen, das Produkt der Formel (I) gegebenenfalls verseift, um ein Produkt der Formel (I) zu erhalten, worin $R_1$ ein Wasserstoffatom bedeutet, die Produkte der Formel (I) gewünschtenfalls der Einwirkung einer Säure oder einer Base unterzieht, um hieraus das Salz zu bilden.

2. Verfahren gemäß Anspruch 1 zur Herstellung der Verbindungen der Formel (I), worin $R_1$, $R_2$ und $R_3$ die vorstehend angegebenen Bedeutungen besitzen und worin n = 1, sowie von deren Additionssalzen mit Säuren und Basen, dadurch gekennzeichnet, daß man eine Säure der Formel (II)

$$R'_1 - \overset{\overset{O}{\parallel}}{C} - S - CH_2 - \underset{\underset{R_2}{|}}{CH} - COOH \quad (II)$$

worin $R'_1$ und $R_2$ die angegebenen Bedeutungen besitzen, oder ein funktionelles Derivat dieser Säure der Einwirkung eines Produkts der Formel (III)

$$H_2N - R_3 \qquad (III)$$

unterzieht, worin $R_3$ die angegebene Bedeutung besitzt, um ein Produkt der Formel (I) zu erhalten, worin $R_1$ =

$$R'_1 - \underset{\underset{O}{\parallel}}{C} - ,$$

$R'_1$ sowie $R_2$ und $R_3$ die angegebenen Bedeutungen besitzen und worin n = 1, das Produkt der Formel (I) gegebenenfalls verseift, um ein Produkt der Formel (I) zu erhalten, worin $R_1$ ein Wasserstoffatom bedeutet, die Produkte der Formel (I) gewünschtenfalls der Einwirkung einer Base oder einer Säure unterzieht, um hieraus das Salz zu bilden.

3. Verfahren gemäß Anspruch 1 zur Herstellung der Verbindungen der Formel (I), worin $R_1$, $R_2$ und $R_3$ die vorstehend angegebenen Bedeutungen besitzen und n eine ganze Zahl von 1 bis 5 darstellt, sowie von deren Additionssalzen mit Säuren und Basen, dadurch gekennzeichnet, daß man eine Säure der Formel (IV)

$$X_1 - (CH_2)_n - \overset{\overset{R_2}{|}}{CH} - COOH \qquad (IV)$$

worin $X_1$ ein Halogenatom darstellt und worin n und $R_2$ die vorstehend angegebenen Bedeutungen besitzen, oder ein funktionelles Derivat derselben der Einwirkung eines Produktes der vorstehenden Formel (III) ($NH_2$-$R_3$) unterzieht, um ein Produkt der Formel (V)

$$X_1 - (CH_2)_n - \overset{\overset{R_2}{|}}{CH} - CONHR_3 \qquad (V)$$

zu erhalten, worin $X_1$, n, $R_2$ und $R_3$ die vorstehenden Bedeutungen besitzen, das man der Einwirkung eines anions einer Thiosäure der Formel VI

$$R'_1 - \underset{\underset{O}{\parallel}}{C} - SH \qquad (VI)$$

unterzieht, worin $R'_1$ die angegebene Bedeutung besitzt, um ein Produkt der Formel (I) zu erhalten, worin $R_1$ =

$$R'_1 - \underset{\underset{O}{\parallel}}{C} - ,$$

n eine ganze Zahl von 1 bis 5 bedeutet und $R'_1$, $R_2$ und $R_3$ die vorstehenden Bedeutungen besitzen, das Produkt der Formel (I) gegebenenfalls verseift, um ein Produkt der Formel (I) zu erhalten, worin $R_1$ ein Wasserstoffatom bedeutet, und die Produkte der Formel (I) gewünschtenfalls der Einwirkung einer Säure oder einer Base unterzieht, um hieraus das Salz zu bilden.

4. Verfahren gemäß Anspruch 1 zur Herstellung von Verbindungen der Formel (I) gemäß Anspruch 1 der Formel (I')

$$R_1 - S - (CH_2)_n - \underset{\displaystyle R_2}{CH} - \overset{\displaystyle O}{\overset{\|}{C}} - NH\ R'_3 \qquad (I')$$

worin $R_1$, $R_2$ und n die vorstehenden Bedeutungen besitzen und $R'_3$ einen heterocyclischen Rest, ausgewählt unter den Thiazolyl-, 4,5-Dihydrothiazolyl-, Pyridinyl-, Oxazolyl-, Isoxazolyl-, Imidazolyl-, Pyrimidyl-, Tetrazolyl-, Benzimidazolyl-Resten, gegebenenfalls substituiert durch einen Alkylrest mit 1 bis 5 Kohlenstoffatomen, bedeutet oder $R'_3$ einen Phenylrest darstellt, der gegebenenfalls durch einen oder mehrere Reste substituiert ist, ausgewählt unter den Alkylresten mit 1 bis 5 Kohlenstoffatomen, den Alkoxyresten mit 1 bis 5 Kohlenstoffatomen, der Hydroxylgruppe, der Nitrogruppe, den Halogenatomen, der Trifluormethylgruppe und den Resten

$$-N{<}^{X}_{X'} ,$$

worin X und X' gleich oder voneinander verschieden sind und ein Wasserstoffatom oder einen Alkylrest mit 1 bis 5 Kohlenstoffatomen bedeuten, wobei $R'_3$ keinen substituierten oder unsubstituierten Phenylrest darstellen kann, wenn $R_2$ ein Wasserstoffatom bedeutet und wenn n gleich 1 ist, sowie von deren Additionssalzen mit Säuren und Basen, dadurch gekennzeichnet, daß man von einem Produkt der Formel (III) ausgeht, worin $R_3$ die vorstehend angegebenen Bedeutungen von $R'_3$ besitzt.

5. Verfahren gemäß Anspruch 4, dadurch gekennzeichnet, daß man von einem Produkt der Formel (II) oder (VI) ausgeht, worin

$$R'_1 - \underset{\displaystyle O}{\underset{\|}{C}} -$$

eine Acetylgruppe darstellt.

6. Verfahren gemäß Anspruch 5, dadurch gekennzeichnet, daß man von einem Produkt der Formel (II) oder (IV) ausgeht, worin $R_2$ einen Benzylrest bedeutet und n für 1 steht.

7. Verfahren gemäß einem der Ansprüche 4 bis 6, dadurch gekennzeichnet, daß man von einem Produkt der Formel (III) ausgeht, worin $R_3$ einen gegebenenfalls durch ein oder mehrere Chloratome oder einen oder mehrere Methoxyreste substituierten Phenylrest oder eine Pyridinylgruppe bedeutet.

8. Verfahren gemäß einem der Ansprüche 1 bis 4, dadurch gekennzeichnet, daß man eines der Produkte der Formel (I) mit den folgenden Bezeichnungen herstellt:

α-(Mercaptomethyl)-N-phenyl-benzolpropanamid,
S-[3-Oxo-3-(phenylamino)-2-(phenylmethyl)-propyl]-ethanthioat,
α-(Mercaptomethyl)-N-(3-methoxyphenyl)-benzol-propanamid,
α-(Mercaptomethyl)-N-(4-methoxyphenyl)-benzol-propanamid,
α-(Mercaptomethyl)-N-(4-pyridinyl)-benzol-propanamid
sowie deren Additionssalze mit Säuren und Basen.

## Claims

for the contracting states: BE CH DE FR GB IT LI LU NL SE

1. Compounds with the formula (I):

$$R_1\text{-}S\text{-}(CH_2)_n\text{-}\underset{\displaystyle R_2}{CH}\text{-}\overset{\displaystyle O}{\overset{\|}{C}}\text{-}NH\text{-}R_3 \qquad (I)$$

in which $R_1$ represents a hydrogen atom or a

$$-\underset{\displaystyle O}{\underset{\|}{C}}-R'_1$$

radical, $R'_1$ being an alkyl radical containing from 1 to 5 carbon atoms or an aryl radical possibly substituted by a hydroxy radical, an alkyl radical containing from 1 to 5 carbon atoms, an alkoxy radical containing from 1

to 5 carbon atoms, a nitro radical or a halogen atom, n represents a whole number able to vary from 1 to 5, $R_2$ represents a hydrogen atom, an alkyl radical containing from 1 to 5 carbon atoms, an aryl or arylalkyl radical containing from 6 to 15 carbon atoms, possibly substituted by an alkyl radical containing from 1 to 5 carbon atoms, an alkoxy radical containing from 1 to 5 carbon atoms, a hydroxyl radical, a halogen atom or a trifluoromethyl radical, $R_3$ represents a heterocyclic radical chosen from the following radicals:- thiazolyl, 4,5-dihydrothiazolyl, pyridinyl, oxazolyl, isoxazolyl, imidazolyl, pyrimidyl, tetrazolyl, benzimidazolyl, benzothiazolyl or benzoxazolyl, possibly substituted by an alkyl radical containing from 1 to 5 carbon atoms or $R_3$ represents a hydrogen atom, a phenyl radical possibly substituted by one or more radicals chosen from the group constituted by alkyl radicals containing from 1 to 5 carbon atoms, alkoxy radicals containing from 1 to 5 carbon atoms, a hydroxyl radical, a nitro radical, halogen atoms, a trifluoromethyl radical, a carboxymethyl radical, alkoxy carbonylmethyl radicals in which the alkoxy radical contains from 1 to 5 carbon atoms, aralkyloxy radicals containing from 7 to 15 carbon atoms and the radicals:

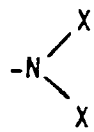 ,

in which X and X', identical or different, represent a hydrogen atom or an alkyl radical containing from 1 to 5 carbon atoms, $R_3$ however not being able to represent a phenyl radical, not substituted or substituted by one or more radicals chosen from the group constituted by alkyl radicals containing from 1 to 5 carbon atoms, alkoxy radicals containing from 1 to 5 carbon atoms, and halogen atoms, when $R_2$ represents a hydrogen atom and when n is equal to 1 or 2, and $R_3$ not being able to represent a phenyl radical substituted by a carboxymethyl radical when $R_2$ represents a methyl radical and when n is equal to 1, as well as their addition salts with acids and bases.

2. Compounds with the formula (I) as defined in claim 1, for which $R_1$ is a hydrogen atom, as well as their addition salts with acids and bases.

3. Compounds with the formula (I) as defined in claim 1, for which $R_1$ is an acetyl radical, as well as their addition salts with acids and bases.

4. Compounds with the formula (I) as defined in any one of the claims 1 to 3, for which $R_2$ does not represent a hydrogen atom, as well as their addition salts with acids and bases.

5. Compounds with the formula (I) as defined in any one of the claims 1 to 4, for which n = 1 and $R_2$ is a benzyl radical, as well as their addition salts with acids and bases.

6. Compounds with the formula (I) as defined in any one of the claims 1 to 5, for which $R_3$ is a phenyl radical possibly substituted by one or more chlorine atoms or by one or more methoxy radicals or a pyridinyl radical, as well as their addition salts with acids and bases.

7. Any one of the compounds with the formula (I) according to claim 1 of which the names follow:

- -(mercaptomethyl)N-phenyl benzene propanamide,
- ethanethioate of S-[3-oxo-3-(phenylamino)2-(phenylmethyl)propyl],
- -(mercaptomethyl)N-(3-methoxyphenyl)benzene propanamide,
- -(mercaptomethyl)N-(4-methoxyphenyl)benzene propanamide,
- -(mercaptomethyl)N-(4-pyridinyl)benzene propanamide,

as well as their addition salts with acids and bases.

8. Preparation process for products with the formula (I) in which $R_1$, $R_2$ and $R_3$ have the previously given significances and in which n = 1, characterized in that an acid with the formula (II):

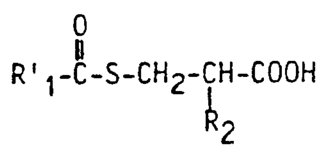 (II)

in which $R'_1$ and $R_2$ have the significances already indicated, or a functional derivative of this acid, is submitted to the action of a product with the formula (III):

$$H_2N - R_3 \qquad (III)$$

in which $R_3$ has the significance already indicated, so as to obtain a product with the formula (I) in which: $R_1$ =

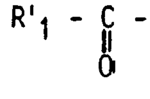 ,

$R'_1$ as well as $R_2$ and $R_3$ have the significances already indicated and in which n = 1, which product with the formula (I), if necessary or desired, is saponified, so as to obtain a product with the formula (I) in which $R_1$ represents a hydrogen atom, which products with the formula (I) are submitted, if

desired, to the action of a base or an acid so as to form the corresponding salts.

9. Preparation process for products with the formula (I) in which $R_1$, $R_2$ and $R_3$ have the previously given significances and in which n represents a whole number able to vary and from 1 to 5, characterized in that an acid with the formula (IV):

$$X_1-(CH_2)_n-\overset{R_2}{\overset{|}{CH}}-COOH \qquad (IV)$$

in which $X_1$ is a halogen atom and in which n and $R_2$ have the significances given above, or a functional derivative of this acid, is submitted to the action of a product with the previous formula (III) ($NH_2$-$R_3$), so as to obtain a product with the formula (V):

$$X_1-(CH_2)_n-\overset{R_2}{\overset{|}{CH}}-CONHR_3 \qquad (V)$$

in which $X_1$, n, $R_2$ and $R_3$ have the previous significances, which is submitted to the action of the anion of a thioacid with the formula (VI):

$$R'_1-\underset{\underset{O}{\|}}{C}-SH \qquad (VI)$$

in which $R'_1$ keeps its significance, so as to obtain a product with the formula (I) in which $R_1$ =

$$= R'_1-\underset{\underset{O}{\|}}{C}-,$$

n represents a whole number able to vary from 1 to 5 and in which $R'_1$, $R_2$ and $R_3$ have the significances given above, which product with the formula (I), if necessary or desired, is saponified, so as to obtain a product with the formula (I) in which $R_1$ represents a hydrogen atom, which products with the formula (I) are submitted, if desired, to the action of an acid or of a base so as to form the corresponding salts.

10. As medicaments, products with the formula (I) as defined in any one of the claims 1 to 6, as well as their addition salts with pharmaceutically acceptable acids or bases.

11. As medicaments, products as defined by claim 7, as well as their addition salts with pharmaceutically acceptable acids and bases.

12. Pharmaceutical compositions containing as active principle, one at least of the medicaments as defined by claim 10 or 11.

13. As new industrial products, products with the formula (V) as defined in claim 9.

## Claims

for the contracting state: AT

1. Process for preparing compounds with the formula (I):

$$R_1-S-(CH_2)_n-\underset{\underset{R_2}{|}}{CH}-\overset{\overset{O}{\|}}{C}-NH-R_3 \qquad (I)$$

in which $R_1$ represents a hydrogen atom or a

$$-\underset{\underset{O}{\|}}{C}-R'_1$$

radical, $R'_1$ being an alkyl radical containing from 1 to 5 carbon atoms or an aryl radical possibly substituted by a hydroxy radical, an alkyl radical containing from 1 to 5 carbon atoms, an alkoxy radical containing from 1 to 5 carbon atoms, a nitro radical or a halogen atom, n represents a whole number able to vary from 1 to 5, $R_2$ represents a hydrogen atom, an alkyl radical containing from 1 to 5 carbon atoms, an aryl or arylalkyl radical containing from 6 to 15 carbon atoms, possibly substituted by an alkyl radical containing from 1 to 5 carbon

atoms, an alkoxy radical containing from 1 to 5 carbon atoms, a hydroxyl radical, a halogen atom or a trifluoromethyl radical, $R_3$ represents a heterocyclic radical chosen from the following radicals:- thiazolyl, 4,5-dihydrothiazolyl, pyridinyl, oxazolyl, isoxazolyl, imidazolyl, pyrimidyl, tetrazolyl, benzimidazolyl, benzothiazolyl or benzoxazolyl, possibly substituted by an alkyl radical containing from 1 to 5 carbon atoms or $R_3$ represents a hydrogen atom, a phenyl radical possibly substituted by one or more radicals chosen from the group constituted by alkyl radicals containing from 1 to 5 carbon atoms, alkoxy radicals containing from 1 to 5 carbon atoms, a hydroxyl radical, a nitro radical, halogen atoms, a trifluoromethyl radical, a carboxymethyl radical, alkoxy carbonylmethyl radicals in which the alkoxy radical contains from 1 to 5 carbon atoms, aralkyloxy radicals containing from 7 to 15 carbon atoms and the radicals:

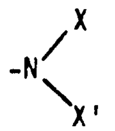 ,

in which X and X', identical or different, represent a hydrogen atom or an alkyl radical containing from 1 to 5 carbon atoms, $R_3$ however not being able to represent a phenyl radical, not substituted or substituted by one or more radicals chosen from the group constituted by alkyl radicals containing from 1 to 5 carbon atoms, alkoxy radicals containing from 1 to 5 carbon atoms, and halogen atoms, when $R_2$ represents a hydrogen atom and when n is equal to 1 or 2, and $R_3$ not being able to represent a phenyl radical substituted by a carboxymethyl radical when $R_2$ represents a methyl radical and when n is equal to 1, as well as their addition salts with acids and bases, characterized in that:

- to prepare compounds with the formula (I) in which $R_1$, $R_2$ and $R_3$ have the previously given significance and in which n = 1, as well as their addition salts with acids and bases, an acid with the formula (II):

$$R'_1-\overset{\overset{O}{\|}}{C}-S-CH_2-\underset{R_2}{\underset{|}{CH}}-COOH \qquad (II)$$

in which $R'_1$ and $R_2$ have the significances already indicated, or a functional derivative of this acid, is submitted to the action of a product with the formula (III):

$$H_2N - R_3 \qquad (III)$$

in which $R_3$ has the significance already indicated, so as to obtain a product with the formula (I) in which:

$$R_1 = R'_1 - \underset{\underset{O}{\|}}{C} -,$$

$R'_1$ as well as $R_2$ and $R_3$ have the significances already indicated and in which n = 1,

which product with the formula (I), if necessary or desired, is saponified, so as to obtain a product with the formula (I) in which $R_1$ represents a hydrogen atom, which products with the formula (I) are submitted, if desired, to the action of a base or an acid so as to form the corresponding salts;

- to prepare compounds with the formula (I) in which $R_1$, $R_2$ and $R_3$ have the previously given significance and in which n is a whole number varying from 1 to 5, as well as their addition salts with acids and bases, an acid with the formula (IV):

$$X_1-(CH_2)_n-\overset{\overset{R_2}{|}}{CH}-COOH \qquad (IV)$$

in which $X_1$ is a halogen atom and in which n and $R_2$ have the significances given above, or a functional derivative of this acid, is submitted to the action of a product with the previous formula (III) ($NH_2$-$R_3$), so as to obtain a product with the formula (V):

$$X_1-(CH_2)_n-\overset{\overset{R_2}{|}}{CH}-CONHR_3 \qquad (V)$$

in which $X_1$, n, $R_2$ and $R_3$ have the previous significances, which is submitted to the action of the anion of a thioacid with the formula (VI):

$$R'_1 - \underset{\overset{\|}{O}}{C} - SH \qquad (VI)$$

in which $R'_1$ keeps its significance, so as to obtain a product with the formula (I) in which $R_1 = R'_1$

$$- \underset{\overset{\|}{O}}{C} -,$$

n represents a whole number able to vary from 1 to 5 and in which $R'_1$, $R_2$ and $R_3$ have the significances given above, which product with the formula (I), if necessary or desired, is saponified, so as to obtain a product with the formula (I) in which $R_1$ represents a hydrogen atom, which products with the formula (I) are submitted, if desired, to the action of an acid or of a base so as to form the corresponding salts.

2. Process according to claim 1, for the preparation of compounds with the formula (I) in which $R_1$, $R_2$ and $R_3$ have the previously given significance and in which n = 1, as well as their salts of addition with acids and bases, characterized in that an acid with the formula (II):

$$R'_1\text{-}\overset{\overset{O}{\|}}{C}\text{-}S\text{-}CH_2\text{-}\underset{\underset{R_2}{|}}{CH}\text{-}COOH \qquad (II)$$

in which $R'_1$ and $R_2$ have the significances already indicated, or a functional derivative of this acid, is submitted to the action of a product with the formula (III):

$$H_2N - R_3 \qquad (III)$$

in which $R_3$ has the significance already indicated, so as to obtain a product with the formula (I) in which: $R_1$ =

$$R'_1 - \underset{\overset{\|}{O}}{C} -,$$

$R'_1$ as well as $R_2$ and $R_3$ have the significances already indicated and in which n = 1, which product with the formula (I), if necessary or desired, is saponified, so as to obtain a product with the formula (I) in which $R_1$ represents a hydrogen atom, which products with the formula (I) are submitted, if desired, to the action of a base or an acid so as to form the corresponding salts.

3. Process according to claim 1, for the preparation of compounds with the formula (I), in which $R_1$, $R_2$ and $R_3$ have the previously given significance and in which n is a whole number varying from 1 to 5, as well as their addition salts with acids and bases, characterized in that an acid with the formula (IV):

$$X_1\text{-}\ (CH_2)_n - \overset{\overset{R_2}{|}}{CH}\ \text{-}COOH \qquad (IV)$$

in which $X_1$ is a halogen atom and in which n and $R_2$ have the significances given above, or a functional derivative of this acid, is submitted to the action of a product with the previous formula (III) ($NH_2$-$R_3$), so as to obtain a product with the formula (V):

$$X_1\text{-}\ (CH_2)_n - \overset{\overset{R_2}{|}}{CH} - CONHR_3 \qquad (V)$$

in which $X_1$, n, $R_2$ and $R_3$ have the previous significances, which is submitted to the action of the anion of a thioacid with the formula (VI):

$$R'_1 - \underset{\overset{\|}{O}}{C} - SH \qquad (VI)$$

in which $R'_1$ keeps its significance, so as to obtain a product with the formula (I) in which $R_1$ =

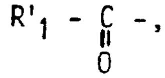

n represents a whole number able to vary from 1 to 5 and in which $R'_1$, $R_2$ and $R_3$ have the significances given above, which product with the formula (I), if necessary or desired, is saponified, so as to obtain a product with the formula (I) in which $R_1$ represents a hydrogen atom, which products with the formula (I) are submitted, if desired, to the action of an acid or of a base so as to form the corresponding salts.

4. Process according to claim 1, for the preparation of compounds with the formula (I) as defined in claim 1, answering to the formula (I'):

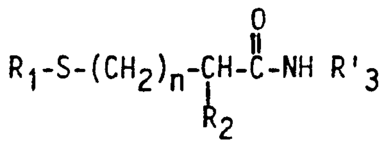 (I')

in which $R_1$, $R_2$ and n keep their previously given significances and $R'_3$ represents a heterocyclic radical chosen from the following radicals:-thiazolyl, 4,5-dihydrothiazolyl, pyridinyl, oxazolyl, isoxazolyl, imidazolyl, pyrimidyl, tetrazolyl, benzimidazolyl, possibly substituted by an alkyl radical containing from 1 to 5 carbon atoms or $R_3$ represents a phenyl radical possibly substituted by one or more radicals chosen from the group constituted by alkyl radicals containing from 1 to 5 carbon atoms, alkoxy radicals containing from 1 to 5 carbon atoms, a hydroxyl radical, a nitro radical, halogen atoms, a trifluoromethyl radical and

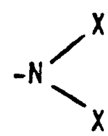

radicals, X and X', identical or different, represent a hydrogen atom, or an alkyl radical containing from 1 to 5 carbon atoms, $R_3$ however not being able to represent a phenyl radical, substituted or not substituted, when $R_2$ represents a hydrogen atom and when n is equal to 1, as well as of their addition salts with acids and bases, characterized in that at the start a product with the formula (III), in which $R_3$ has the values of $R'_3$ indicated above, is used.

5) Process according to claim 4, characterized in that at the start a product with the formula (II) or (VI), in which

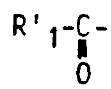

represents an acetyl radical, is used.

6) Process according to claim 5, characterized in that at the start a product with the formula (II) or (IV), in which $R_2$ represents a benzyl radical and n is equal to 1, is used.

7) Process according to any one of the claims 4 to 6, characterized in that at the start a product with the formula (III), in which $R_3$ is a phenyl radical possibly substituted by one or more chlorine atoms or by one or more methoxy radicals or a pyridinyl radical, is used.

8) Process according to any one of the claims 1 to 4, characterized in that any one of the products with the formula (I) is prepared, of which the names follow:

- -(mercaptomethyl)N-phenyl benzene propanamide,
- ethanetnioate of S-[3-oxo-3-(phenylamino)2-(phenylmethyl)propyl],
- -(mercaptomethyl)N-(3-methoxyphenyl)benzene propanamide,
- -(mercaptomethyl)N-(4-methoxyphenyl)benzene propanamide,
- -(mercaptomethyl)N-(4-pyridinyl)benzene propanamide,

as well as their addition salts with acids and bases.